# EUROPEAN PATENT SPECIFICATION

(11) **EP 1 084 269 B1**
(45) Date of publication and mention of the grant of the patent: **25.04.2007**
(21) Application number: 98928880.8
(22) Date of filing: 04.06.1998
(51) Int. Cl.: C12Q 1/28, C12Q 1/00, A01N 43/16, C12Q 1/26, C07D 295/18

(54) **NOVEL PEROXIDASE SUBSTRATES AND THEIR USE IN CATALYZED REPORTER DEPOSITION**
NEUARTIGE PEROXIDASESUBSTRATE UND IHRE ANWENDUNG IN KATALYSIERTEN REPORTERBINDUNGSASSAYS
NOUVEAUX SUBSTRATS DE PEROXYDASE ET LEUR UTILISATION DANS LE DEPOT CATALYSE D'ENZYMES REPORTEURS

(43) Date of publication of application: 21.03.2001
(73) Proprietor: PerkinElmer LAS, Inc., Waltham, MA 02451 (US)
(72) Inventor: BOBROW, Mark, Norman, Lexington, MA 02421 (US)
(74) Representative: Horner, Martin Grenville
(86) International application number: PCT/US1998/011477
(87) International publication number: WO 1999/063108

(56) References cited:
- EP-A- 0 097 096
- US-A- 4 876 084
- US-A- 4 978 523
- US-A- 5 583 001
- BOBROW M N ET AL: "CATALYZED REPORTER DEPOSITION, A NOVEL METHOD OF SIGNAL AMPLIFICATION II. APPLICATION TO MEMBRANE IMMUNOASSAYS" JOURNAL OF IMMUNOLOGICAL METHODS, ELSEVIER SCIENCE PUBLISHERS B.V.,AMSTERDAM, NL, vol. 137, no. 1, 1 March 1991 (1991-03-01), pages 103-112, XP002045236 ISSN: 0022-1759
- CEVASCO G.; THEA S.: 'Participation of an extended p-oxo ketene intermediate in the dissociative alkaline hydrolysis of aryl 4-hydroxycinnamates' JOURNAL OF ORGANIC CHEMISTRY vol. 59, 1994, pages 6274 - 6278
- CEVASCO G.; THEA S.: 'The dissociative hydrolysis of 2-4 dinitrophenyl 4 hydroxycinnamate' GAZETTA CHIMICA ITALIANA vol. 122, 1992, pages 199 - 200

## Description

### Field of the Invention

This invention relates to novel peroxidase substrates, and more particularly, to cinnamoyl-containing substrates which can be used in a variety of applications such as catalyzed reporter deposition.

### Background of the Invention

Peroxidase, because of its high turnover rate, good stability, and availability is widely used in enzyme-based analytical methods. For example, horseradish peroxidase (HRP) (EC 1.11.1.7) catalyzes the oxidation of a large variety of hydrogen-donating substrates with hydrogen peroxide. HRP is also one of the preferred enzymes for use in catalyzed reporter deposition.

Catalyzed reporter deposition is a novel method of signal amplification which constitutes the subject matter of U.S. Patent Nos. 5, 196,306 and 5,583,001. It is also discussed in Bobrow et al., Journal of Immunological Methods, 125: 279-285 (1989) and in Bobrow et al., Journal of Immunological Methods, 137: 103-112 (1991).

The method utilizes an analyte-dependent enzyme activation ("ADEAS") to catalyze the deposition of additional reporter enzyme onto the solid phase, resulting in signal amplification and improved assay detection limits. In a preferred embodiment, HRP is the ADEAS.

The ADEAS reacts with a conjugate consisting of a detectably labeled substrate specific for theADEAS. When the ADEAS and the conjugate react, an activated conjugate is formed which deposits covalently wherever receptor for the activated conjugate is immobilized. The receptor is not reactive with the analyte-dependent enzyme activation system.

Conjugates can be synthesized using conventional coupling and labeling techniques. Substrate choice will depend upon the ADEAS selected. Thus, detailed knowledge is required of the catalytic properties of each specific enzyme in order to properly design a useful synthetic substrate and, if necessary, a receptor. Examples of conjugates which have been described include substituted phenols such as biotin tyramine, fluorescein tyramine, NADP, phosphorylated biotin, etc.

However, care must be exercised in designing substrates suitable for peroxidase-mediated assays. Guilbault et al., Analytical Chemistry, Vol. 40, No. 8, pages 1256 - 1263 (1968) studied a wide variety substrates for fluorometric determination of oxidative enzymes such as peroxidase, galactose oxidase, glucose oxidase, and invertase. These researchers reported that 3,4-dihydroxycinnamic acid, which contains a double bond in the side chain was not a useful substrate for the fluorometric determination of oxidative enzymes because it stopped the reaction with the enzyme.

US-A-4,978,523 relates to a melanin inhibitor comprising a reactive component of a cinnamic acid derivative which may be applied to affected portions such as freckles and pigmentory deposits after sunburn without giving any stimulative or allergic troubles to the skin.

EP-A-0 097 096 relates to a method for preparing phenolamides which have an antiviral and a hormonal biological activity.

Bobrow et al.:"Catalysed reporter deposition, a novel method of signal amplification II. Application to membrane immunoassays" Journal of Immunological Methods, Elsevier Science Publishers B.V., Amsterdam, ML. Vol. 137, No. 1 relates to the utilisation of amplification for nonradiometric membrane assays where detection is facilitated by the formation of an insoluble chromogenic product.

US-A-5,583,001 relates to a method to catalyse reporter deposition to improve detection or quantitation of an analyte in a sample by amplifying the detector signal which comprises reacting an analyte dependent enzyme activation system with a conjugate consisting of a detectably labeled substrate specific for the enzyme system, said conjugate reacts with the analyte dependent enzyme activation system to form an activated conjugate which deposits substantially wherever receptor for the activated conjugate is immobilised, said receptor not being reactive with the analyte dependent activation system.

Cevasco G. and Thea S.: "Participation of an extended p-oxo ketene intermediate in the dissociative alkaline hydrolysis of aryl 4-hydroxycinnamates" Journal of Organic Chemistry, vol. 59, 1994, pages 6274 - 6278 relates to the alkaline hydrolysis of 4-hydroxycinnamate esters of acidic phenols.

Cevasco G and Thea S.: "The dissociative hydrolysis of 2-4 dinitrophenyl 4 hydroxycinnamate" Gazetta Chimica Italiana, vol. 122, 1992, pages 199 - 200 relates to the dissociative hydrolysis of 2,4-dinitrophenyl 4-hydroxycinnamate.

According to a first aspect of the present invention there is provided a method for the detection or quantitation of an analyte in an assay which comprises the following steps:
a) reacting an analyte dependent enzyme activation system comprising at least one enzyme with a conjugate comprising a detectably labelled substrate having the structure of a p-hydroxycinnamoyl-containing compound having the structure: 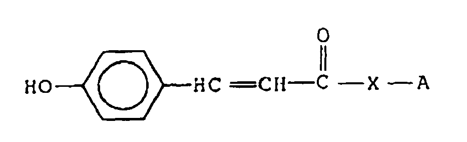 wherein:
   X is a linker group, capable of linking A to a p-hydroxycinnamoyl moiety, and
   A is a label which is detectable in an analyte-dependent enzyme activation system assay and is selected from the group consisting of biotin, dinitrophenyl, fluorescein and tetramethylrhodamine.to form an activated conjugate which covalently deposits substantially wherever there is at least one receptor for the activated conjugate, said receptor not being reactive with the analyte dependent enzyme activation system; and
b) detecting or quantitating signals which are generated directly or indirectly from the deposited detectable labels.

According to a second aspect of the present invention there is provided a p-hydroxycinnamoyl-containing compound having the structure: 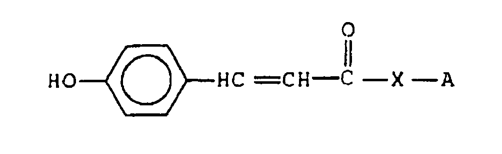 wherein:
X is a linker group selected from the group consisting of: 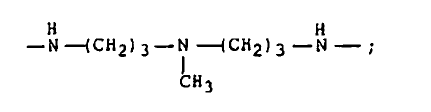 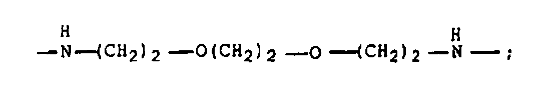 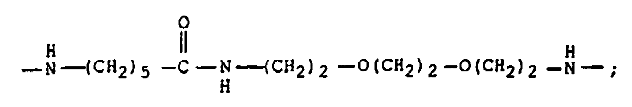 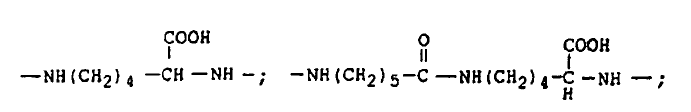 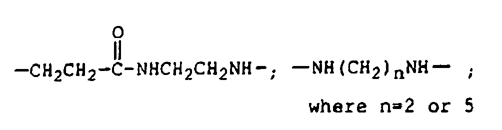 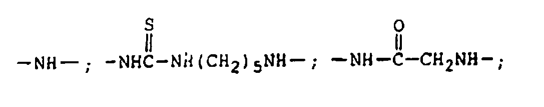 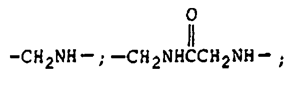 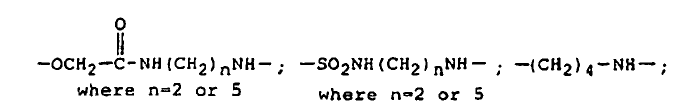 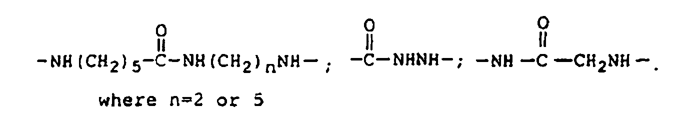 wherein X is capable of linking A to a p-hydroxycinnamoyl moiety, and
A is a label which is detectable in an analyte-dependent enzyme activation system assay and is selected from the group consisting of biotin, dinitrophenyl, fluorescein and tetramethylrhodamine.

### Brief Description of the Figures

Figure 1 shows detection of cytomegalovirus early antigen using catalyzed reporter deposition to amplify the detector signal. HRP was the ADEAS and biotin tyramide was the conjugate.
Figure 2 shows detection of cytomegalovirus early antigen using catalyzed reporter deposition to amplify the detector signal. HRP was the ADEAS and N-1-biotinyl-N-2-(4-hydroxycinnamoyl)-ethane diamine was the conjugate. This conjugate was used at a ten-fold lower concentration than biotin tyramide.

### Detailed Description of the Invention

The term analyte dependent enzyme activation system (ADEAS) refers to an enzyme system wherein (i) at least one enzyme is coupled, in any manner known to those skilled in the art, to a member of a specific binding pair, or (ii) the enzyme need not be coupled to a member of a specific binding pair when it is the analyte. The enzyme, either by itself or in connection with a second enzyme, catalyzes the formation of an activated conjugate which then is deposited wherever there is a receptor for the activated conjugate.

The term amplification as used herein means amplification of reporter signal due to deposition of a conjugate activated by an ADEAS.

The term conjugate as used herein means a detectably labeled cinnamoyl-containing substrate specific for a peroxidase-mediated ADEAS whether it be a single enzyme ADEAS or multi-enzyme ADEAS conjugate as is discussed below.

The term detectably labeled means that the substrate can be coupled to either a reporter or to an unlabeled first member of a specific binding pair provided that the reporter introduces a different moiety to the substrate as is discussed below. When the substrate is coupled to an unlabeled member of a specific binding pair, following binding to the receptor, the substrate-specific binding partner complex is reacted with the second member of the binding pair which is coupled to a reporter. Alternately, the substrate-specific binding partner complex can be pre-reacted with the detectably labeled other member of the specific binding pair prior to deposition.

The term deposition means directed binding of an activated conjugate to the receptor which results from the formation of a specific binding pair interaction as described below.

The term receptor means a site which will bind to the activated conjugate through the formation of a specific binding pair interaction as described below.

The term activated conjugate means that the conjugate has been primed by the ADEAS to bind with the receptor.

The ADEAS catalyzes deposition of a conjugate (i.e., a detectably labeled substrate specific for the ADEAS) by converting the substrate portion of the conjugate to an activated form which binds covalently wherever there is a receptor. The ADEAS does not utilize enzyme cascade reactions or enzyme cycling to effect amplification. Rather, it uses either a single enzyme or combination of enzymes to activate the conjugate. Deposition of conjugate occurs only if the analyte and ADEAS have been reacted and a receptor is available to bind the activated conjugate. Thus, the ADEAS, conjugate and receptor are chosen to form an operational trio. The analyte and the ADEAS can be the same if the analyte is an enzyme (e.g., peroxidase) or different.

The instant invention concerns novel cinnamoyl-containing substrates which heretofore were thought to be unsuitable substrates for peroxidase-mediated reactions because it would be expected that such cinnamoyl-containing substrates would inhibit the reaction with the enzyme.

ADEAS' suitable for use with the cinnamoyl-containing substrates of the invention include oxidoreductases. More particularly, peroxidases can be mentioned. The preferred ADEAS which is suitable for the novel substrates of the invention is horseradish peroxidase.

It has been found, surprisingly and unexpectedly, that a novel conjugate having a p-hydroxycinnamoyl moiety in its structure significantly improves the sensitivity of catalyzed reporter deposition at least ten-fold beyond the level currently achieved using non-cinnamoyl containing conjugates.

The novel p-hydroxycinnamoyl-containing compounds of the invention have the structure defined in claim 11.

Examples of linker groups attached to detectable labels include the following:

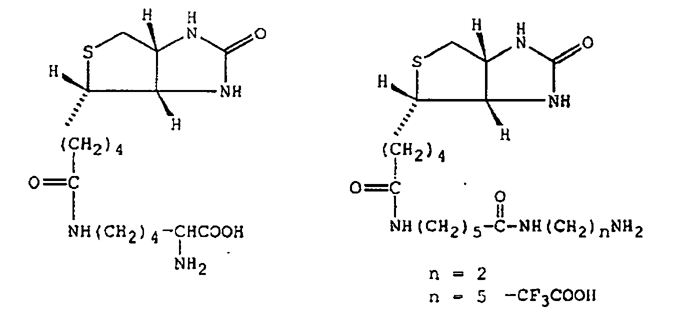

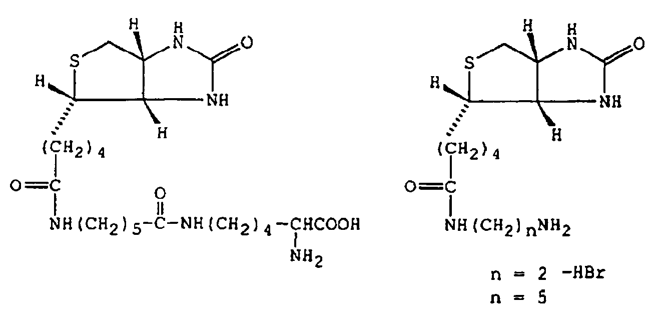

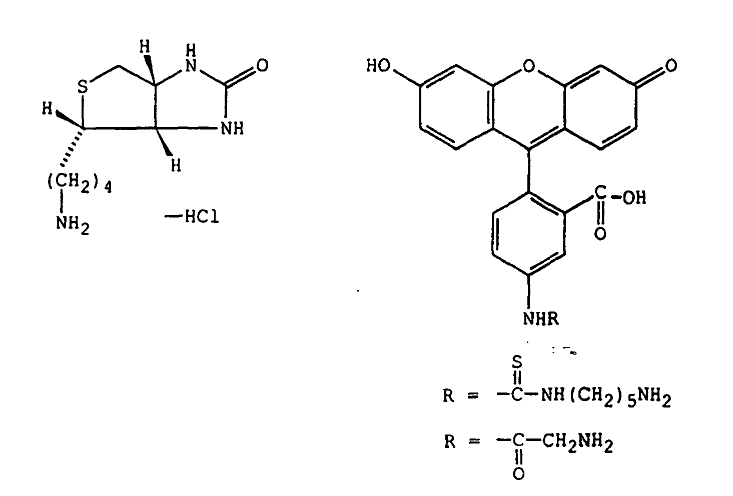

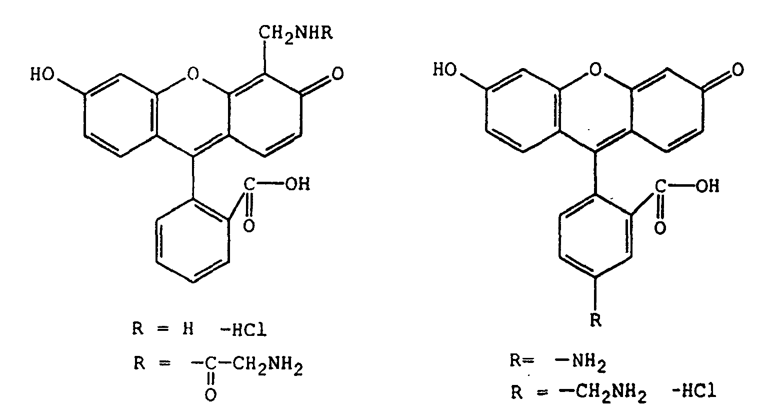

A wide variety of reporters are available for coupling to the substrate to produce the conjugate or to couple to a member of a specific binding pair. Reporters can be a radioactive isotope such as ¹²⁵I, enzymes, fluorogenic, chemiluminescent, or electrochemical materials.

Examples of reporter enzymes which can be used to practice the invention include hydrolases, lyases, oxidoreductases, transferases, isomerases and ligases. Some preferred examples are phosphatases, esterases, glycosidases and peroxidases. Specific examples include alkaline phosphatase, lipases, beta-galactosidase and horseradish peroxidase. As was noted above, if an enzyme is used as a reporter, it can be the same as or different from the enzyme or enzymes used in the ADEAS. The present invention can be used to catalyze deposition of a radioisotopically labeled conjugate or an enzyme-labeled conjugate, etc.

If the reporter is a member of a specific binding pair, then it can be of the immune or the non-immune type. Immune specific binding pairs are exemplified by antigen/antibody systems or hapten/antihapten systems. The antibody member, whether polyclonal, monoclonal or an immunoreactive fragment thereof, can be produced by customary methods familiar to those skilled in the art. The terms immunoreactive antibody fragment or immunoreactive fragment mean fragments which contain the binding region of the antibody. Such fragments may be Fab-type fragments which are defined as fragments devoid of the Fc portion, e.g., Fab, Fab' and F(ab')₂ frag,emts, or may be so-called "half-molecule" fragments obtained by reductive cleavage of the disulfide bonds connecting the heavy chain components of the intact antibody. If the antigen member of the specific binding pair is not immunogenic, e.g., a hapten, it can be covalently coupled to a carrier protein to render it immunogenic.

Non-immune binding pairs include systems wherein the two components share a natural affinity for each other but are not antibodies. Exemplary non-immune binding pairs are biotin-avidin or biotin-streptavidin, folic acid-folate binding protein, complementary probe nucleic acids, etc.

The compounds of the invention can be synthesized using conventional coupling and labeling techniques as illustrated in the examples below. One approach can be to react p-hydroxycinnamic acid according to the following scheme:

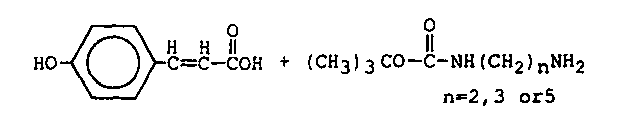

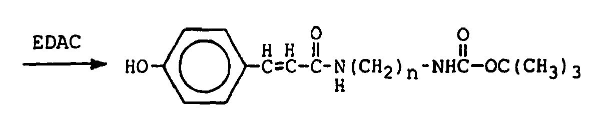

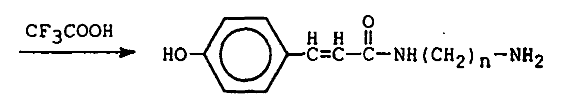

The resulting amine can then be reacted with suitable labels containing N-hydroxysuccinimide or isothiocyanate moieties to produce the final p-hydroxycinnamoyl-containing compound.

As was noted above, many linker groups attached to detectable labels are commercially available. These commercially available linker groups can be reacted with p-hydroxycinnamic acid using conventional protocols well known to those skilled in the art.

When a detectably labeled p-hydroxycinnamoyl-containing compound (conjugate) of the invention is activated, it will bind wherever there is a receptor. The activated conjugate binds to the receptor via a specific binding pair interaction which in this case is a covalent bond. An exogenous receptor means a receptor which does not originate within the assay. It can be immobilized on the surface of a support prior to adding the conjugate to the reaction mixture. An endogenous receptor means a receptor which originates within the assay. It is believed that the novel conjugates of the invention, when activated, bind with receptors suitable for activated phenolic moieties such as electron rich moieties.

In another embodiment, this invention concerns the use of these detectably labeled cinnamoyl-containing compounds in assays for detecting or quantitating the presence or absence of an analyte in a sample using catalyzed reporter deposition to amplify the reporter signal. Virtually any assay format such as an immunoassay or a nucleic acid hybridization assay can be used.

It should be clear to those skilled in the art that a large number of variations are possible and all these variations fall within the scope of the invention.

The following examples are intended to illustrate the invention and should not be construed as limitations thereon.

### EXAMPLE 1

### 4-Hydroxycinnamic acid, N-hydroxysuccinimide ester

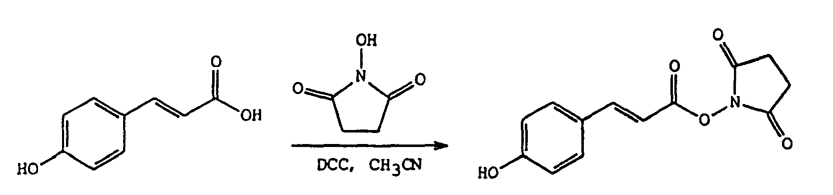

p-Hydroxycinnamic acid (5.0 g, 30.5 mmol) is dissolved in acetonitrile (150 mL) with warming followed by the addition of N-hydroxysuccinimide (4.0 g, 35 mmol) and 1,3-dicyclohexylcarbodiimide (1 M in dichloromethane, 35 mL, 35 mmol). The pale yellow solution is stirred at room temperature overnight, filtered, and the filtrate is evaporated and purified by silica gel chromatography, eluting with 1:1 ethyl acetate:hexane. Fractions containing product by thin layer chromatography are pooled and evaporated to give a white solid (3.31 g, 42%). ¹H NMR (DMSO-d₆) δ 7.9 (d, 1H, vinyl), 7.7 (d, 2H, phenyl 3,5), 6.9 (d, 2H, phenyl 2,6), 6.7 (d, 1H, vinyl), 2.8 (s, 4H, succinimide methylene).

### EXAMPLE 2

### N-1-Biotinyl-N-2-(4-hydroxycinnamoyl)-ethane diamine

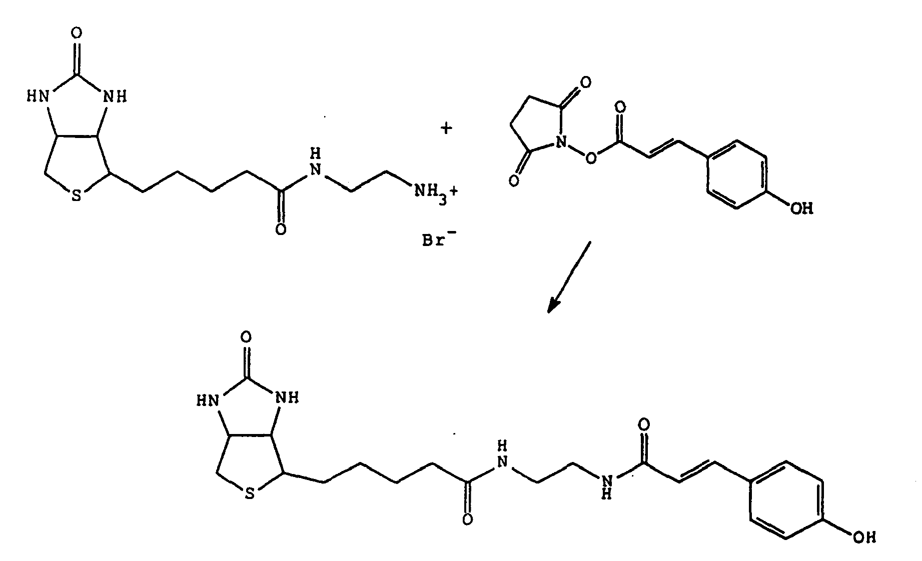

N-(2-aminoethyl)biotinamide hydrobromide (25 mg, 0.068 mmol), triethylamine (10 µL, 0.072 mmol), and 4-hydroxycinnamic acid, succinimidyl ester (18 mg, 0.068 mmol) are dissolved in dimethylformamide (DMF) (1 mL) and stirred overnight at room temperature. The reaction mixture is purified by C 18 reverse phase chromatography, eluting with a linear gradient of 0-100% methanol in water over 30 minutes. The peak eluting at 22 minutes is collected and evaporated to give 22 mg of a white solid (0.051 mmol, 75%). ¹H NMR (CD₃OD) δ 7.5 (d, 1H, vinyl), 7.4 (d, 2H, aromatic 3,5), 6.8 (d, 2H, aromatic 2,6), 6.4 (d, 1H, vinyl), 4.4 (m, 1H, biotin methine-NH), 4.2 (m, 1H, biotin methine-NH), 3.4 (m, 4H, ethane diamide), 3.1 (m, 1H, biotin methine-S), 2.8 (1H, biotin methylene-S), 2.6 (1H, biotin methylene-S), 2.2 (2H, biotin CH₂-CO), 1.3-1.7 (m, 6H, biotin aliphatics).

### EXAMPLE 3

### N-ε-(2,4-Dinitrophenyl)-N-α-(4-Hydroxycinnamoyl)-L-Lysine

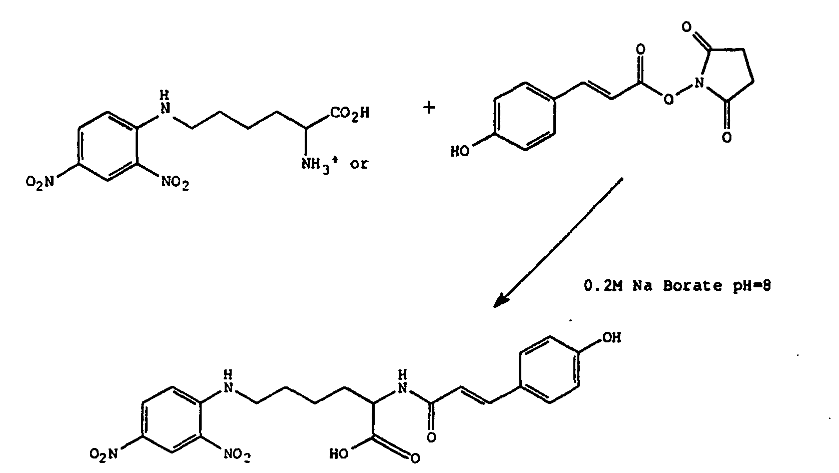

N-ε-(2,4-Dinitrophenyl)-L-lysine (32 mg, 0.09 mmol) is suspended in 0.2 M sodium borate (3 mL) pH=8, and a solution of 4-hydroxycinnamic acid, succinimidyl ester (35 mg, 0.13 mmol) in DMF (0.5 mL) is added in small portions with stirring. The yellow suspension is stirred at room temperature overnight and the crude reaction mixture is purified by C 18 reverse phase chromatography, eluting with a linear gradient of 50-100% methanol in 50 mM sodium phosphate, pH=3 over 15 minutes. The major peak eluting at approximately 11 minutes is pooled from several injections, desalted on a C8 sep-pak cartridge, and evaporated to give 10 mg of a yellow solid (0.02 mmol, 25%). ¹H NMR (CD₃OD) δ 8.9 (s, 1H, DNP H-3), 8.2 (d, 1H, DNP H-5), 7.4 (d, 1 H, vinyl), 7.3 (d, 2H), 7.2 (d, 1H, DNP H-6), 6.8 (d, 2H, aromatic 2,6), 6.4 (d, 1H, vinyl), 4.5 (m, 1H, lysine methine), 3.5 (t, 2H, CH₂-NH), 2.1-1.5 (m, 6H, CH₂).

### EXAMPLE 4

### DAMP-Cinnamate

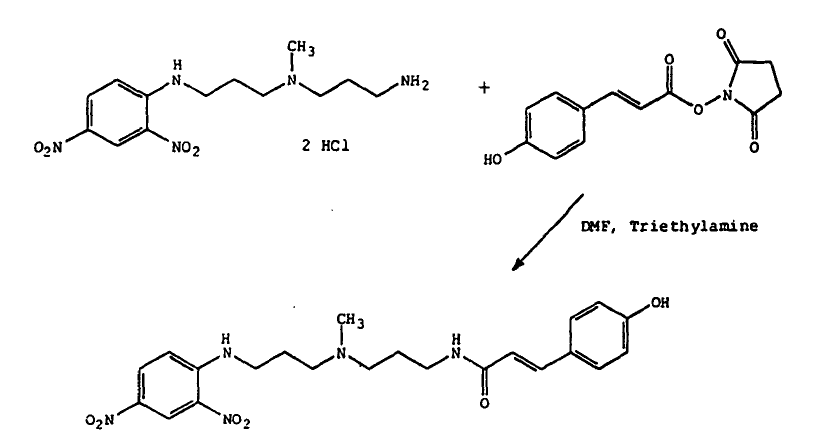

N-(3-((2,4-Dinitrophenyl)amino)propyl)-N-(3-aminopropyl)methylamine, dihydrochloride (DAMP) (45 mg, 0.12 mmol), triethylamine (35 µL, 0.25 mmol), and 4-hydroxycinnamic acid, succinimidyl ester (35 mg, 0.13 mmol) are dissolved in dimethylformamide (DMF) (1 mL) and stirred overnight at room temperature. The reaction mixture is evaporated in vacuo, dissolved in a minimum amount of medthanol/water, and purified by C8 reverse phase chromatography, eluting with a linear gradient of 0-100% methanol in water over 30 minutes. The first major peak is collected and evaporated to give 27 mg of a yellow solid (0.06 mmol, 49%). ¹H NMR (CD₃OD) δ 8.9 (s, 1H, DNP H-3), 8.2 (d, 1H, DNP H-5), 7.4 (d, 1H, vinyl), 7.3 (d, 2H), 7.2 (d, 1H, DNP H-6), 6.8 (d, 2H, aromatic 2,6), 6.4 (1, 1H, vinyl), 3.6 (t, 2H, CH₂-NH), 3.4 (t, 2H, CH₂-NH), 3.3 (t, 2H, CH₂-NH), 3.2 (t, 2H, CH₂-NH), 2.9 (s, 3H, methyl), 2.2-1.9 (m, 4H, CH₂).

### EXAMPLE 5

### N-α-(4-Hydroxycinnamoyl)-L-Lysine

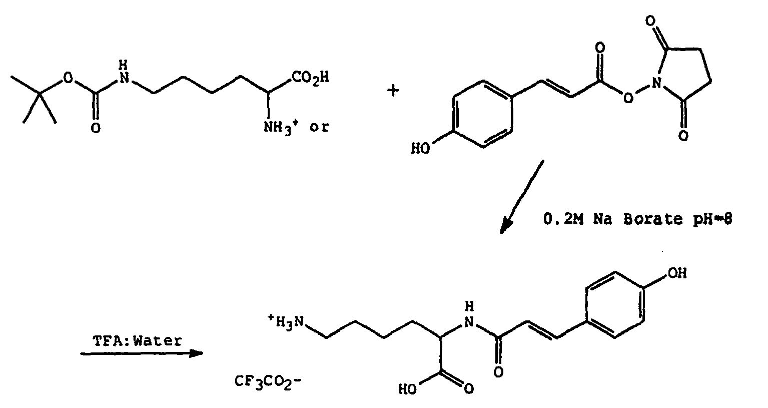

N-ε-BOC-L-lysine hydrochloride (78 mg, 0.32 mmol) is suspended in 0.2 M sodium borate, pH=8 and a solution of 4-hydroxycinnamic acid, succinimidyl ester (130 mg, 0.50 mmol) in DMF (3 mL) is added in small portions with stirring. The light yellow solution is stirred at room temperature overnight and the crude reaction mixture is purified by C18 reverse phase chromatography, eluting with a linear gradient of 0-100% methanol in 50 mM sodium phosphate, pH=3.5 over 30 minutes. The major peak eluting at approximately 25 minutes is pooled from several injections and evaporated to give a glassy solid which is deprotected by stirring with trifluoroacetic acid:water (7:3) (10 mL) for 24 hours. The solution is evaporated in vacuo, stripped from toluene (3x10 mL), and triturated with ether to give 97 mg of an off white glassy solid (0.24 mmol, 75%).

### EXAMPLE 6

### N-ε-(5-(and-6)-Carboxyfluoresceinyl)-N-α-(4-Hydroxycinnamoyl)-L-Lysine

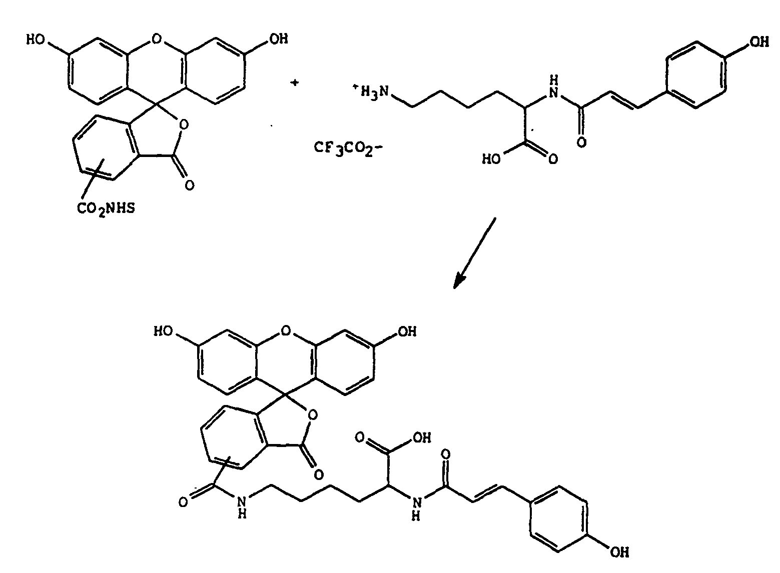

N-α-(4-Hydroxycinnamoyl)-L-lysine (24 mg, 0.06 mmol) is dissolved in water (1 mL) and the pH is adjusted to 9-10 by adding 1N NaOH. 5-(and-6)Carboxyfluorescein, N-hydroxysuccinimide ester (39 mg, 0.08 mmol) is added as a solid in several portions and the reaction mixture is stirred overnight at room temperature and purified by C18 reverse phase chromatography, eluting with a linear gradient of 0-100% methanol in 50 mM sodium phosphate, pH=3.5 over 30 minutes. The major peak is pooled from several injections to give a yellow solution containing 0.06 mmol (100%) of the desired product.

### EXAMPLE 7

### Detection of Cytomegalovirus (CMV) Early Antigen

CMV infected cells on a slide (eight well slides with MRC-5 cells infected with CMV, Hemagen Diagnostics, Inc.) were hydrated with phosphate buffered saline (PBS) for two minutes. An anti-CMV-horseradish peroxidase conjugate (prepared by a modification of the method of Ishikawa, E., et al., J. Immunoassay, 4, 209-237, 1983) was diluted in 0.1M tris, 0.15M NaCl, 0.5% milk protein, pH 7.5 and incubated on the slide at 37°C for 30 minutes. The slide was then washed with 0.1M tris, 0.15M NaCl, 0.05% tween 20, pH 7.5 (TNT) buffer for two minutes. Biotinyl-tyramide (NEN Life Science Products, NEL-700) was diluted to 2 µg/ml in Amplification Diluent (NEN Life Science Products, NEL-700), added to one of the wells of the slide and incubated for 10 minutes at room temperature. N-1-Biotinyl-N-2-(4-hydroxycinnamoyl)-ethane diamine was diluted to 0.2 µg/ml in Amplification Diluent, added to a second well of the slide and incubated for 10 minutes at room temperature. The slide was washed two times for five minutes each with TNT buffer. Streptavidin-fluorescein (NEN Life Science Products, NEL-720) was diluted 1:500 in TNT buffer and incubated for 30 minutes at room temperature. The slide was washed three times for five minutes each with TNT buffer. Evans Blue (0.001%) was incubated on the slide for 1 minute, the slide was rinsed two times with deionized water and air dried. After applying anti-fade mounting media (Vectashield) and a cover slip, the slide was imaged on a Zeiss fluorescence microscope at 200X magnification.

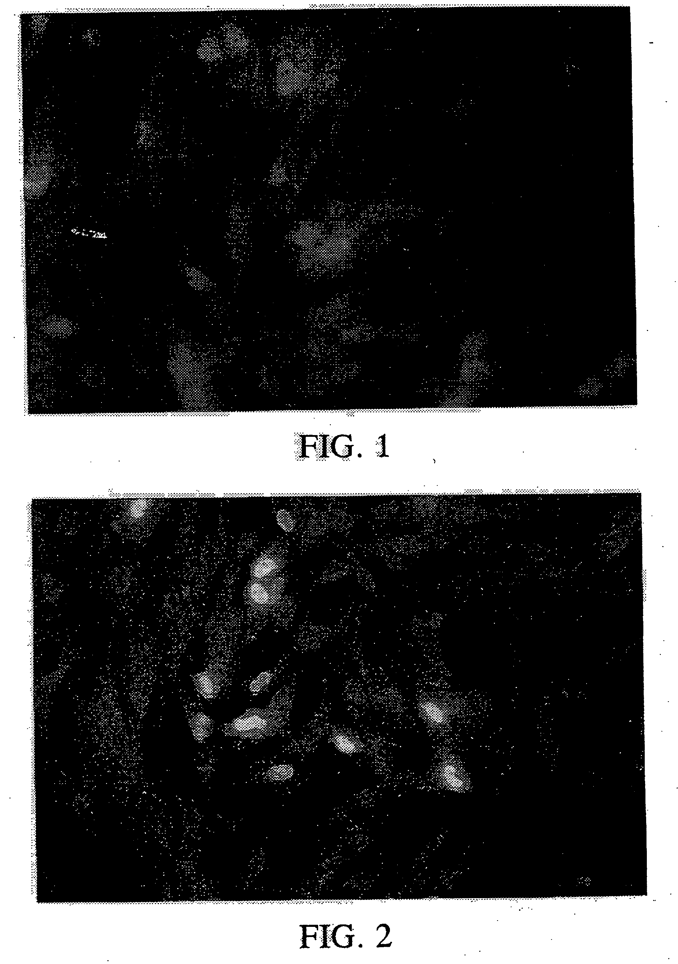

Results: Figure 2 demonstrates that using the substrate, N-1-Biotinyl-N-2-(4-hydroxycinnamoyl)-ethane diamine, at a ten fold lower concentration than biotinyl-tyramide results in far superior sensitivity as evidenced by the bright CMV infected nuclei. It is so sensitive, that endogenous peroxidase activity not detected by the biotinyl-tyramide amplification, is clearly detected using N-1-Biotinyl-N-2-(4-hydroxycinnamoyl)-ethane diamine. In routine practice, an endogenous peroxidase inactivation step is utilized, however, this step was not performed for this example in order to further illustrate the superior sensitivity obtained using N-1-Biotinyl-N-2(4-hydroxycinnamoyl)-ethane diamine.

## Claims

1. A method for the detection or quantitation of an analyte in an assay which comprises the following steps:
a) reacting an analyte dependent enzyme activation system comprising at least one enzyme with a conjugate comprising a detectably labelled substrate having the structure of a p-hydroxycinnamoyl-containing compound having the structure: 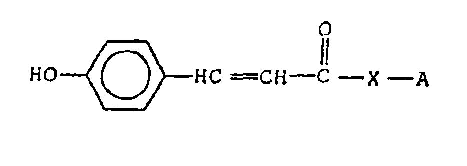 wherein:
X is a linker group, capable of linking A to a p-hydroxycinnamoyl moiety, and
A is a label which is detectable in an analyte-dependent enzyme activation system assay and is selected from the group consisting of biotin, dinitrophenyl, fluorescein and tetramethylrhodamine, to form an activated conjugate which covalently deposits substantially wherever there is at least one receptor for the activated conjugate, said receptor not being reactive with the analyte dependent enzyme activation system; and
b) detecting or quantitating signals which are generated directly or indirectly from the deposited detectable labels.

2. A method according to claim 1, wherein X is selected from the group consisting of:

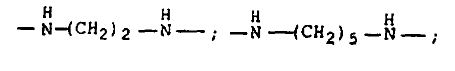

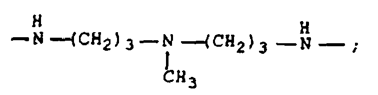

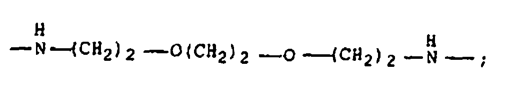

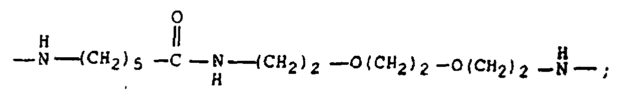

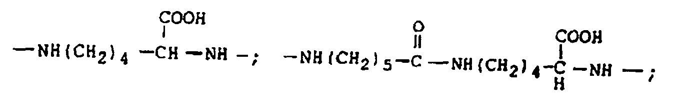

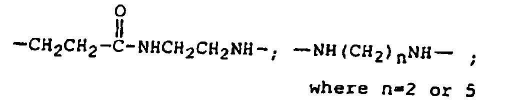

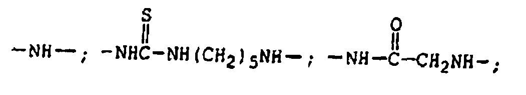

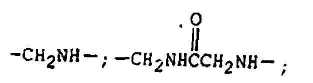

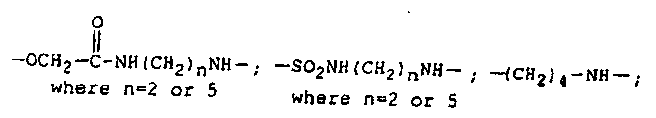

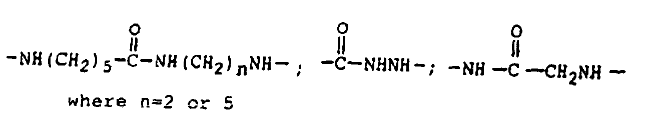

3. A method according to claims 1 or 2, wherein the analyte dependent enzyme activation system is a peroxidase.

4. A method according to claim 1, wherein the assay undergoes the initial step:
immobilizing the analyte on a solid phase to form
the analyte dependent activation system; and wherein the activated conjugate is a first member of a specific binding pair and deposits covalently on the solid phase by binding to a second member of the specific binding pair, and wherein the activated conjugate is not reactive with the analyte dependent enzyme activation system.

5. A method according to claim 4, wherein X is selected from the group consisting of:

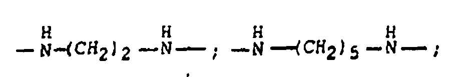

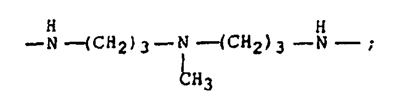

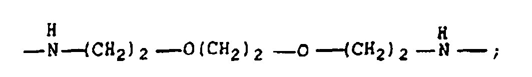

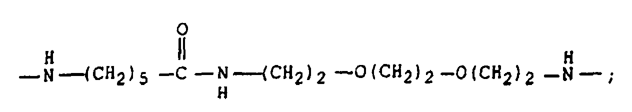

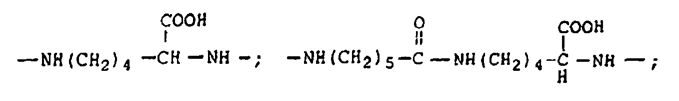

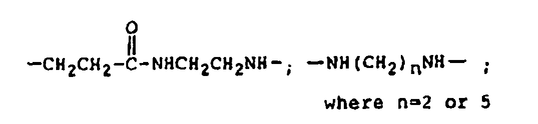

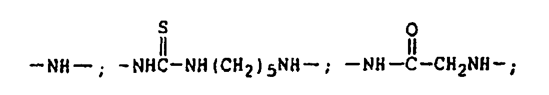

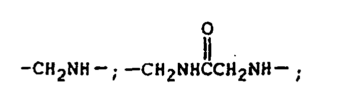

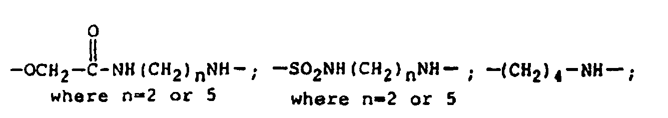

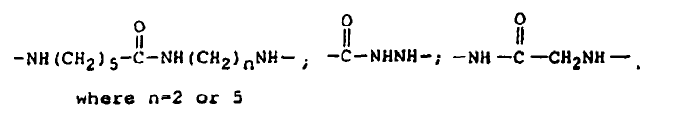

6. A method according to claims 4 or 5, wherein the analyte dependent enzyme activation system is a peroxidase.

7. A method according to claim 1, wherein the analyte-dependent enzyme activation system is a member of a specific binding pair and forms the activated conjugate which is a first member of a specific binding pair, wherein the activated conjugate deposits on the solid phase by binding to a second member of the specific binding pair on the surface of the solid phase, said second member not being reactive with the analyte dependent enzyme activation system.

8. A method according to claim 7, wherein X is selected from the group consisting of:

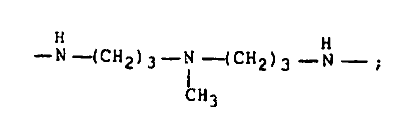

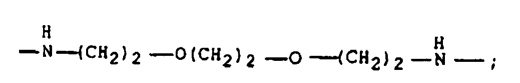

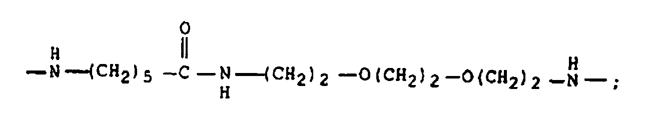

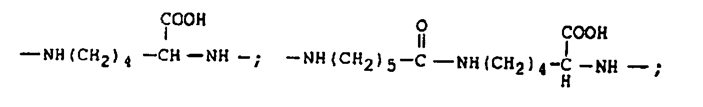

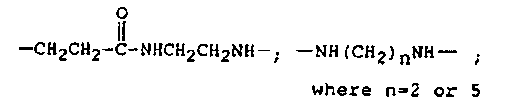

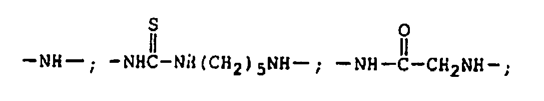

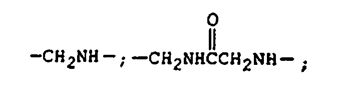

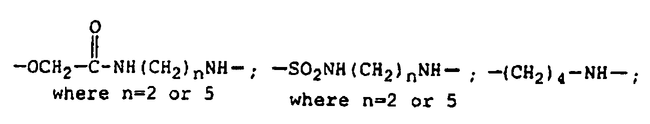

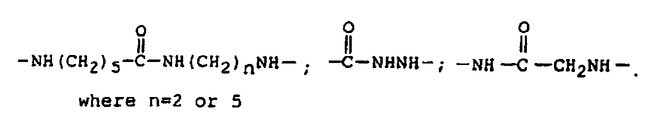

9. A method according to claims 7 or 8, wherein the analyte dependent enzyme activation system is a peroxidase.

10. A method according to claim 1, wherein said at least one enzyme is at least a peroxidase enzyme.

11. A p-hydroxycinnamoyl-containing compound having the structure: 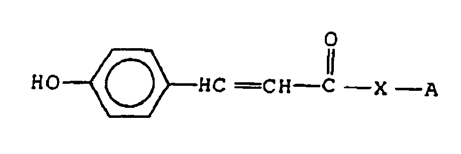 wherein:
X is a linker group selected from the group consisting of: 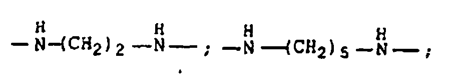 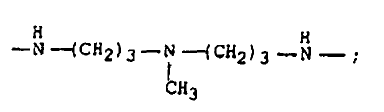 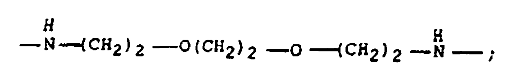 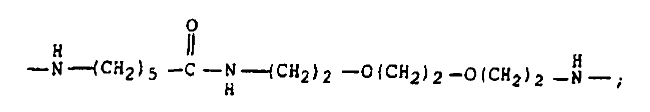 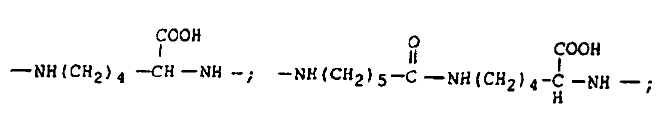 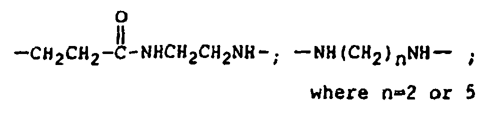 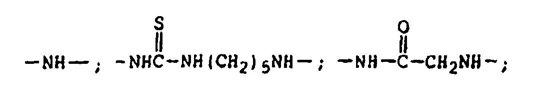 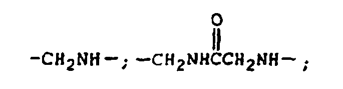 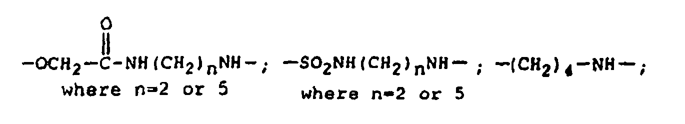 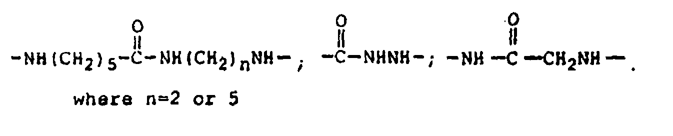 wherein X is capable of linking A to a p-hydroxycinnamoyl moiety, and
A is a label which is detectable in an analyte-dependent enzyme activation system assay and is selected from the group consisting of biotin, dinitrophenyl, fluorescein and tetramethylrhodamine.

## Patentansprüche

1. Verfahren zur Detektierung oder Quantifizierung eines Analyten in einem Assay, der die folgenden Schritte umfasst:
a) Umsetzen eines analytabbängigen Enzymaktivierungssystems, das mindestens ein Enzym umfasst, mit einem Konjugat, das ein detektierbar markiertes Substrat mit der Struktur einer p-Hydroxycinnamoyl enthaltenden Verbindung umfasst, die die folgende Struktur hat: 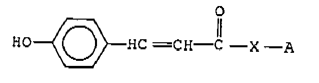 worin:
X eine Linkergruppe ist, die A an eine p-Hydroxycinnamoyleinheit linken kann, und
A eine Markierung ist, die in einem analytabhängigen Enzymaktivierungssystemassay detektierbar ist und ausgewählt ist aus der Gruppe bestehend aus Biotin, Dinitrophenyl, Fluoreszein und Tetramethylrhodamin, um ein aktiviertes Konjugat zu bilden, das sich im Wesentlichen dort kovalent abscheidet, wo sich mindestens ein Rezeptor für das aktivierte Konjugat befindet, wobei der Rezeptor mit dem analytabhängigen Enzymaktivierungssystem nicht reaktiv ist, und
b) Detektieren oder Quantifizieren der Signale, die direkt oder indirekt aus den abgeschiedenen detektierbaren Markierungen erzeugt worden sind.

2. Verfahren nach Anspruch 1, wobei X ausgewählt ist aus der Gruppe bestehend aus:

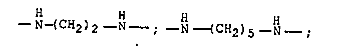

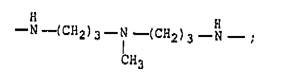

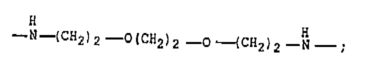

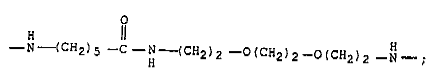

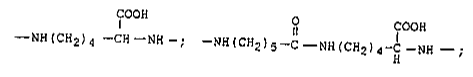

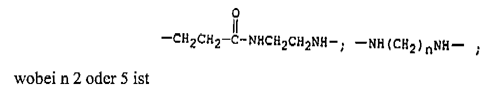

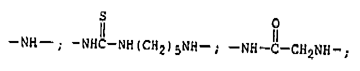

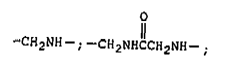

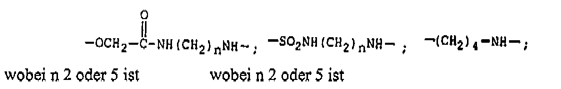

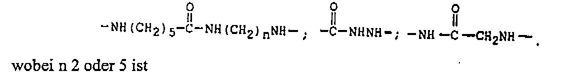

3. Verfahren nach den Ansprüchen 1 oder 2, wobei das analytabhängige Enzymaktivierungssystem eine Peroxidase ist.

4. Verfahren nach Anspruch 1, wobei der Assay den folgenden Anfangsschritt aufweist:
Immobilisieren des Analyten auf einer festen Phase, um das analytabhängige Aktivierungssystem zu bilden, und wobei das aktivierte Konjugat ein erstes Mitglied eines spezifischen Bindungspaars ist und sich kovalent auf der festen Phase abscheidet, indem es an ein zweites Mitglied des spezifischen Bindungspaars bindet, und wobei das aktivierte Konjugat mit dem analytabhängigen Enzymaktivierungssystem nicht reaktiv ist.

5. Verfahren nach Anspruch 4, wobei X ausgewählt ist aus der Gruppe bestehend aus:

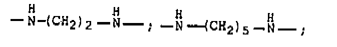

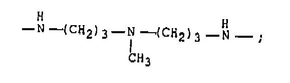

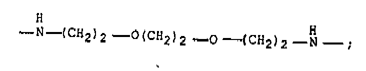

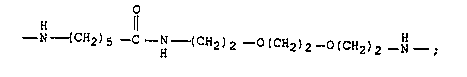

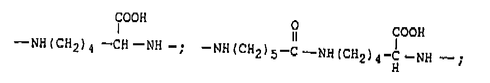

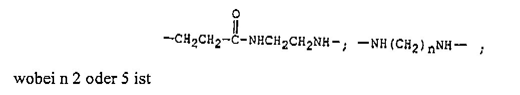

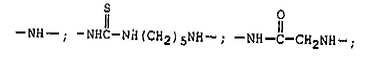

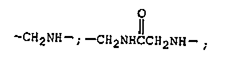

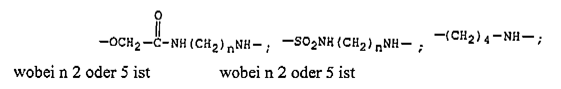

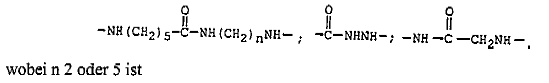

6. Verfahren nach den Ansprüchen 4 oder 5, wobei das analytabhängige Enzymaktivierungssystem eine Peroxidase ist.

7. Verfahren nach Anspruch 1, wobei das analytabhängige Enzymaktivierungssystem ein Mitglied eines spezifischen Bindungspaars ist und das aktivierte Konjugat bildet, welches ein erstes Mitglied eines spezifischen Bindungspaars ist, wobei das aktivierte Konjugat sich auf der festen Phase abscheidet, indem es sich an ein zweites Mitglied des spezifischen Bindungspaars auf der Oberfläche der festen Phase bindet, wobei das zweite Mitglied mit dem analytabhängigen Enzymaktivierungssystem nicht reaktiv ist.

8. Verfahren nach Anspruch 7, wobei X ausgewählt ist aus der Gruppe bestehend aus:

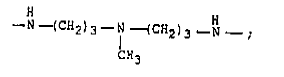

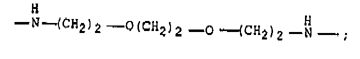

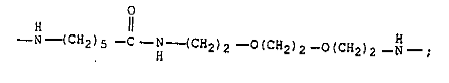

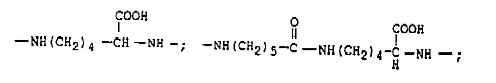

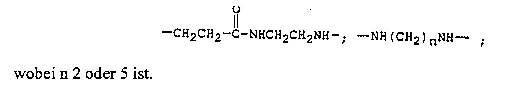

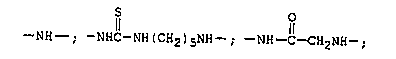

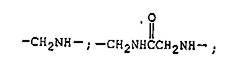

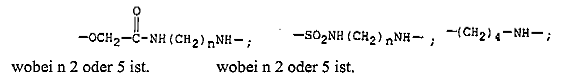

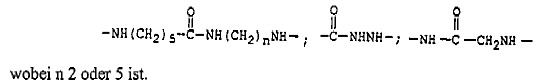

9. Verfahren nach den Ansprüchen 7 oder 8, wobei das analytabhängige Enzymaktivlerungssystem eine Peroxidase ist.

10. Verfahren nach Anspruch 1, wobei das mindestens eine Enzym mindestens ein Peroxidaseenzym ist.

11. p-Hydroxycinnamoyl enthaltende Verbindung mit der folgenden Struktur: 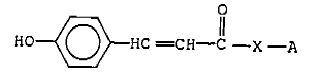 worin:
X eine Linkergruppe ist, die ausgewählt ist aus der Gruppe bestehend aus: 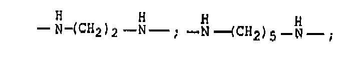 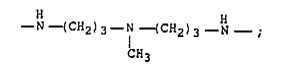 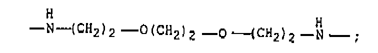 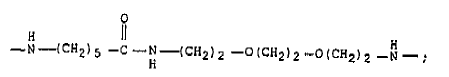 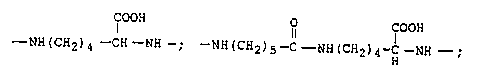 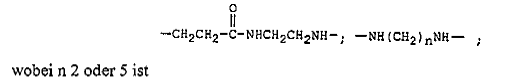 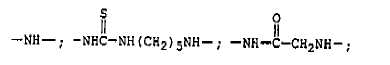 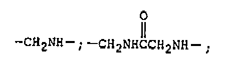 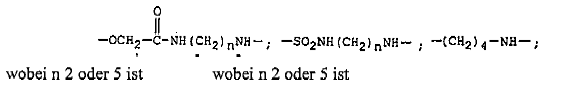 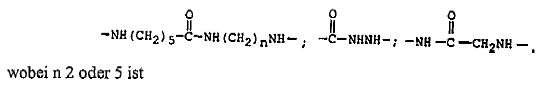 wobei X in der Lage ist, A an eine p-Hydroxycinnamoyleinheit zu linken, und
A eine Markierung ist, die in einem analytabhängigen Enzymaktivierungssystemassay detektierbar ist und ausgewählt ist aus der Gruppe bestehend aus Biotin, Dinitrophenyl, Fluoreszein und Tetramethylrhodamin.

## Revendications

1. Procédé de détection et de quantification d'un analyte dans un test qui comprend les étapes suivantes:
a) la mise en réaction d'un système d'activation enzymatique dépendant d'un analyte comprenant au moins une enzyme avec un conjugué comprenant un substrat marqué de manière détectable ayant la structure d'un composé contenant du p-hydroxycinnamoyl ayant la structure: 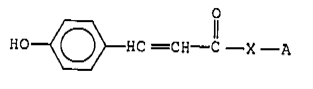 où:
X est un groupe de liaison, capable de lier A à une partie p-hydroxycinnamoyl, et
A est un marqueur qui est détectable dans un test par système d'activation enzymatique dépendant d'un analyte et est sélectionné dans le groupe constitué de biotine, de dinitrophényl, de fluorescéine, et de tétraméthylrhodamine, pour former un conjugué activé qui se dépose de manière covalente sensiblement là où il y a au moins un récepteur du conjugué activé, ledit récepteur ne réagissant pas au système d'activation enzymatique dépendant d'un analyte; et
b) la détection ou la quantification de signaux qui sont produits directement ou indirectement par les marqueurs détectables déposés.

2. Procédé selon la revendication 1, dans lequel X est sélectionné dans le groupe constitué de:

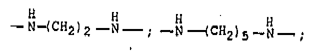

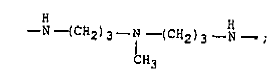

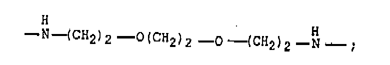

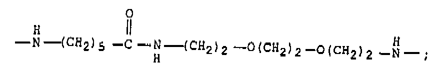

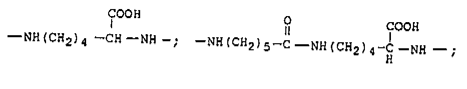

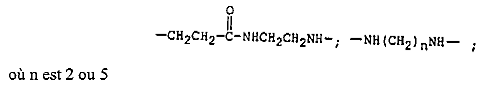

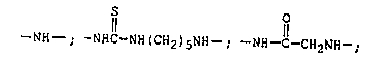

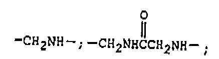

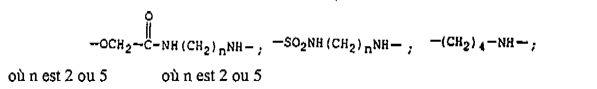

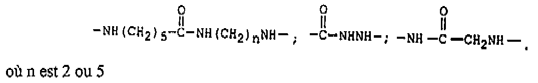

3. Procédé selon les revendications 1 ou 2, dans lequel le système d'activation enzymatique dépendant d'un analyte est une péroxydase.

4. Procédé selon la revendication 1, dans lequel le test suit l'étape initiale:
d'immobilisation de l'analyte sur une phase solide pour former le système d'activation dépendant d'un analyte; et où le conjugué activé est un premier membre d'une paire de liaison spécifique et se dépose de manière covalente sur la phase solide par liaison à un second membre de la paire de liaison spécifique, et où le conjugué activé ne réagit pas avec le système d'activation enzymatique dépendant d'un analyte.

5. Procédé selon la revendication 4, dans lequel X est sélectionné dans le groupe constitué de:

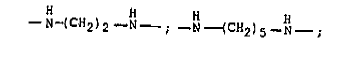

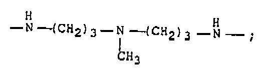

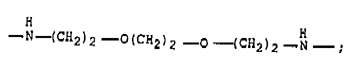

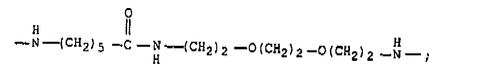

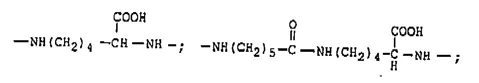

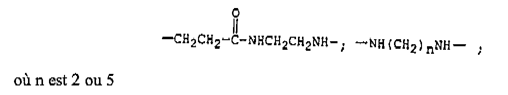

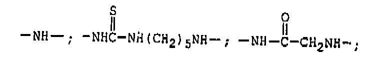

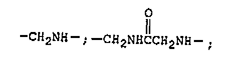

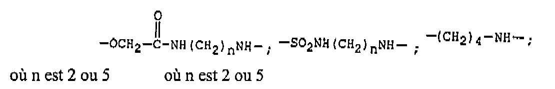

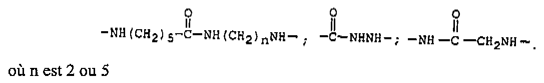

6. Procédé selon les revendications 4 ou 5, dans lequel le système d'activation enzymatique dépendant d'un analyte est une péroxydase.

7. Procédé selon la revendication 1, dans lequel le système d'activation enzymatique dépendant d'un analyte est un membre d'une paire de liaison spécifique et forme le conjugué activé qui est un premier membre d'une paire de liaison spécifique, où le conjugué activé se dépose sur la phase solide par liaison à un second membre de la paire de liaison spécifique sur la surface de la phase solide, ledit second membre ne réagissant pas avec le système d'activation enzymatique dépendant d'un analyte.

8. Procédé selon la revendication 7, dans lequel X est sélectionné dans le groupe constitué de:

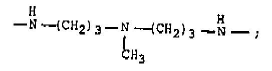

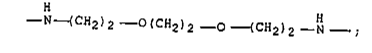

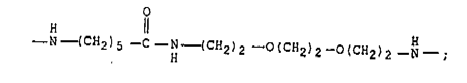

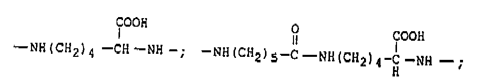

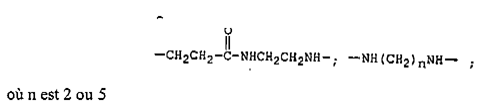

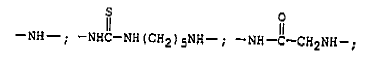

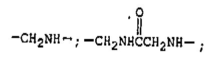

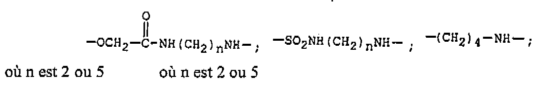

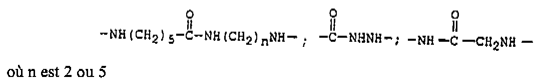

9. Procédé selon les revendications 7 ou 8, dans lequel le système d'activation enzymatique dépendant d'un analyte est une péroxydase.

10. Procédé selon la revendication 1, dans lequel ladite au moins une enzyme est au moins une enzyme péroxydase.

11. Composé contenant du p-hydroxycinnamoyl ayant la structure: 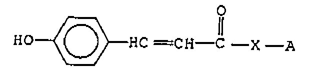 où X est un groupe de liaison sélectionné dans le groupe constitué de: 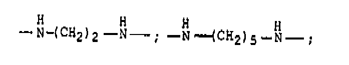 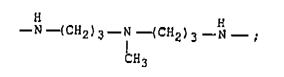 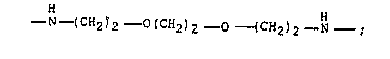 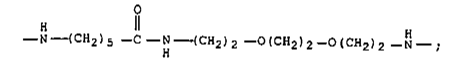 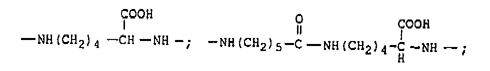 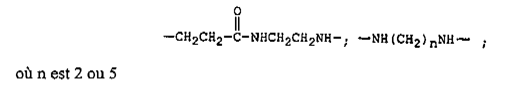 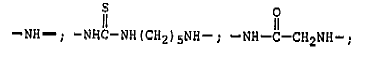 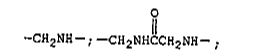 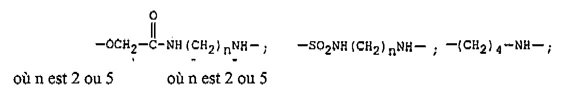 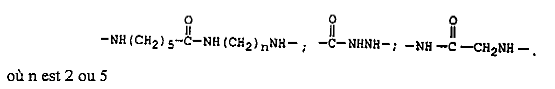 où X est capable de lier A à une partie p-hydroxycinnamoyl, et
A est un marqueur qui est détectable dans un test par système d'activation enzymatique dépendant d'un analyte et est sélectionné dans le groupe constitué de biotine, de dinitrophényl, de fluorescéine, et de tétraméthylrhodamine.
